# EUROPEAN PATENT APPLICATION

(11) **EP 4 494 661 A1**
(43) Date of publication of application: **22.01.2025**
(21) Application number: 24176949.6
(22) Date of filing: 21.05.2024
(51) Int. Cl.: A61L 2/07, A61L 2/24, F26B 5/04, G01N 7/00

(54) **DRYING DETERMINATION METHOD FOR STEAM STERILIZER**

(30) Priority: 21.07.2023 CN 202310897385
(71) Applicant: Ningbo Haishu YESON Medical Device Co., Ltd., 315181 Ningbo Zhejiang (CN)
(72) Inventor: LU, Lihui, Ningbo, 315181 (CN); SHEN, Yuanye, Ningbo, 315181 (CN); ZHENG, Kejie, Ningbo, 315181 (CN)
(74) Representative: Cleanthous, Marinos

(57) **Abstract**

The present disclosure provides a drying determination method for a steam sterilizer, including the following steps: vacuumizing a sterilization chamber by a vacuumizing system; when the sterilization chamber reaches a set vacuum degree, closing a pipeline connecting the sterilization chamber to the vacuumizing system; maintaining the sterilization chamber in vacuum for certain time; during the maintaining the sterilization chamber in vacuum for certain time, continuously detecting a pressure in the sterilization chamber by a pressure detection device; when the pressure detection device detects that the pressure in the sterilization chamber has increased by a value less than P during the period of time, determining that drying is completed; when the pressure detection device detects that the pressure in the sterilization chamber has increased by a value not less than P during the period of time Tl, determining that drying is not completed; opening the pipeline connecting the sterilization chamber to the vacuumizing system again, continuing to vacuumize the sterilization chamber for certain time; and then returning to a drying detection stage until it is determined that drying is completed. The method can accurately determine whether a load is completely dried and then terminate the drying process at appropriate time, thus prolonging the service life of the machine.

## Description

### TECHNICAL FIELD

The present disclosure relates to the technical field of sterilizers, and specifically, to a drying determination method for a steam sterilizer.

### BACKGROUND

Medical devices need to be placed in a steam sterilizer for sterilization and disinfection after use to maintain their cleanliness and prevent cross infection among patients. A sterilization process of the steam sterilizer usually includes the following steps: First, air in a sterilization chamber is extracted through a vacuumizing system of the steam sterilizer, and then a steam system introduces high-temperature and high-pressure steam into the sterilization chamber and keeps the temperature for a period of time to achieve a sterilization effect. After the sterilization ends, the air and water steam in the sterilization chamber are discharged through a water and air discharge system, and then the sterilization chamber is vacuumized through the vacuumizing system. During vacuumizing, the boiling point of water will decrease, making the water easier to vaporize and drain. After the vacuumizing is performed for continuous set time, a load will be gradually dried. Finally, the vacuumizing system is turned off, and an air inlet system is turned on to allow filtered air to enter the sterilization chamber. The drying process ends.

However, due to different types, quantities, initial states, and placement modes of loads to be sterilized, the unified preset drying time cannot guarantee that each load can be dried completely every time. Presetting sufficiently long drying time will cause low sterilization efficiency and a waste of time.

### SUMMARY

The technical problems to be solved by the present disclosure are to overcome the above defects in the prior art and to provide a drying determination method for a steam sterilizer, which can determine whether a load is dried and automatically control drying time according to different loads and different quantities.

A technical solution of the present disclosure provides a drying determination method for a steam sterilizer, including the following steps:
step 1, in a drying stage, vacuumizing a sterilization chamber by a vacuumizing system, and when the sterilization chamber reaches a set vacuum degree, closing a pipeline connecting the sterilization chamber to the vacuumizing system;
step 2, in a drying detection stage, maintaining the sterilization chamber in vacuum for certain time T1, and during the maintaining the sterilization chamber in vacuum for certain time, continuously detecting a pressure in the sterilization chamber by a pressure detection device; and
step 3, in a drying determination stage, when the pressure detection device detects that the pressure in the sterilization chamber has increased by a value less than P during the period of time T 1, determining that drying is completed, and turning off the vacuumizing system;
when the pressure detection device detects that the pressure in the sterilization chamber has increased by a value not less than P during the period of time T1, determining that drying is not completed; opening the pipeline connecting the sterilization chamber to the vacuumizing system again, continuing to vacuumize the sterilization chamber for certain time T2; and then returning to step 2 until it is determined that drying is completed.

As a preference, the pressure detection device in step 2 includes a controller and a pressure sensor; the controller is electrically connected to the pressure sensor; the sterilization chamber is provided with an interface communicated to an inner cavity of the sterilization chamber; and the pressure sensor is connected to the interface.

As a preference, a range of T1 in step 2 is 20 s to 2 min.

As a preference, a range of T2 in step 3 is 1 min to 5 min.

As a preference, a value of P in step 3 is a specific value within a range of 0.6 kPa to 2 kPa.

By the use of the above method, compared with the prior art, the drying determination method for the steam sterilizer of the present disclosure has the following advantages:

In the drying determination stage, the pressure detection device may continuously detect whether the pressure in the sterilization chamber changes. If a load is not completely dried, in a vacuum state, water may be evaporated into water steam and then affect the pressure of the sterilization chamber. The controller may determine that drying is not completed, and step 2 is executed. A load will only be determined to be completely dried after the pressure in the sterilization chamber does not change. The method can accurately determine whether a load is completely dried and then terminate the drying process at appropriate time. It can ensure that the load is completely dried and avoid prolonging the working time of the vacuumizing system, thereby prolonging the service life of the machine.

### BRIEF DESCRIPTION OF THE DRAWINGS

FIG. 1 is a flowchart of the present disclosure.
FIG. 2 is a schematic structural diagram of the present disclosure.
FIG. 3 is a schematic diagram of a mainstream working process of a steam sterilizer.

Descriptions of reference numerals:
1: sterilization chamber; 2: vacuumizing system; 3: water and air discharge system; 4: steam system; 5: pressure detection device; 6: air inlet system; and 7: heating system.

### DETAILED DESCRIPTION OF THE EMBODIMENTS

The present disclosure will be further described in detail below with reference to the accompanying drawings and specific embodiments.

In the descriptions of the present disclosure, it should be understood that orientations or positional relationships indicated by the terms "upper", "lower", "front", "rear", "left", "right", "vertical", "horizontal", "top", "bottom", "inner", "outer", and the like are orientations or positional relationships as shown in the drawings, and are only for the purpose of facilitating and simplifying the descriptions of the present disclosure instead of indicating or implying that devices or elements indicated must have particular orientations, and be constructed and operated in the particular orientations, so that these terms are not construed as limiting the present disclosure. Meanwhile, the terms "first", "second", and the like are only for the purpose of distinguishing the names of the various components and do not have a primary and secondary relationship. Therefore, these terms cannot be understood as limiting the present disclosure.

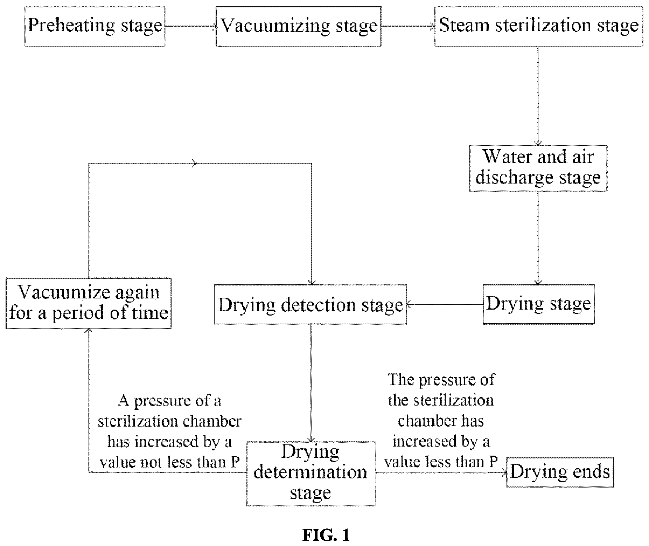

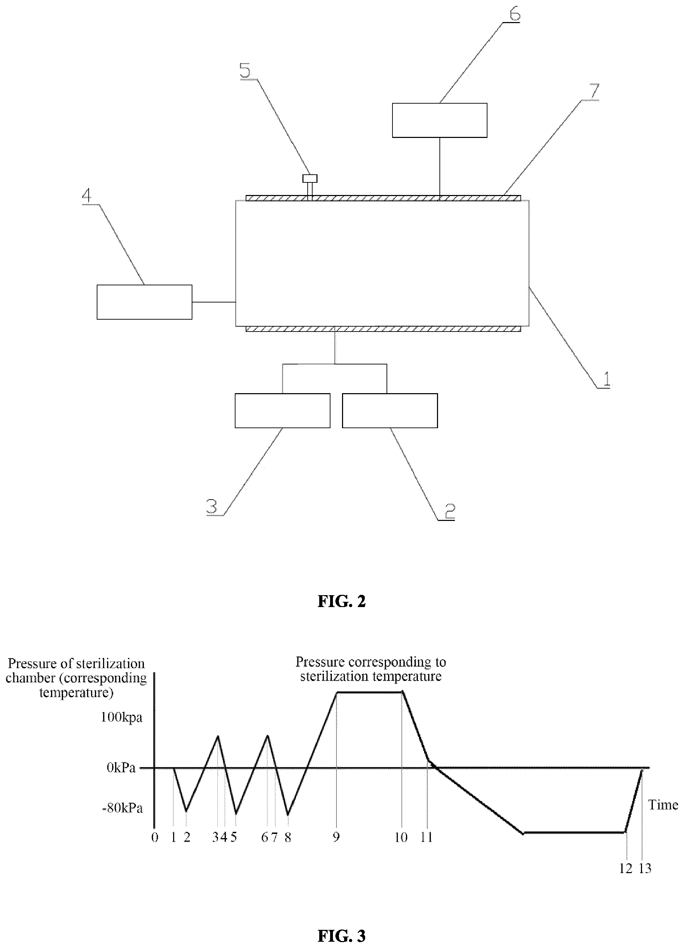

As shown in FIG. 1 and FIG. 2,
the present disclosure discloses a drying determination method for a steam sterilizer, including the following steps:
step 1, in a preheating stage, a heating system preheats an outer wall of a sterilization chamber and controls the sterilization chamber at a certain temperature until a temperature inside the sterilization chamber reaches a preset temperature;
step 2, in a vacuumizing stage, a vacuumizing system vacuumizes the sterilization chamber; a steam system then introduces high-temperature water steam into the sterilization chamber; finally, the vacuumizing system vacuumizes the sterilization chamber again;
step 3, in a steam sterilization stage, the steam system introduces high-temperature steam into the sterilization chamber and maintains the temperature for a period of time to achieve a sterilization effect;
step 4, in a drainage and exhaust stage, a water and air discharge system is turned on to discharge the water and water steam in the sterilization chamber;
step 5, in a drying stage, the vacuumizing system vacuumizes the sterilization chamber, and when the sterilization chamber reaches a set vacuum degree, a pipeline connecting the sterilization chamber to the vacuumizing system is closed;
step 6, in a drying detection stage, the sterilization chamber is maintained in vacuum for certain time T1, and during the maintaining the sterilization chamber in vacuum for certain time, a pressure detection device continuously detects a pressure in the sterilization chamber; and
step 7, in a drying determination stage, when the pressure detection device detects that the pressure in the sterilization chamber has increased by a value less than P during the period of time T1, it is determined that drying is completed, and the vacuumizing system is turned off;
when the pressure detection device detects that the pressure in the sterilization chamber has increased by a value not less than P during the period of time T1, it is determined that drying is not completed; the pipeline connecting the sterilization chamber to the vacuumizing system is opened again; the sterilization chamber is continued to be vacuumized for certain time T2; and then step 6 is executed until it is determined that drying is completed.

A mainstream working process of the steam sterilizer is as shown in FIG. 3: In FIG. 3, 0-1 stands for the preheating stage; 1-8 stands for the vacuumizing stage; 8-10 stands for the sterilization stage; 10-11 stands for the water and air discharge stage; 11-12 stands for the drying stage; and 12-13 stands for an air inlet balancing stage.

The present disclosure adds the drying determination process based on an original working flow of the steam sterilizer. In the drying determination stage, the pressure detection device may continuously detect whether the pressure in the sterilization chamber changes. If a load is not completely dried, in a vacuum state, water may be evaporated into water steam and then affect the pressure of the sterilization chamber. The controller may determine that drying is not completed. The pipeline connecting the sterilization chamber to the vacuumizing system is opened again to continue to vacuumize the sterilization chamber for the time T2, and step 6 is executed. A load will only be determined to be completely dried after the pressure in the sterilization chamber does not change. The method can accurately determine whether a load is completely dried and then terminate the drying process at appropriate time. It can ensure that the load is completely dried and avoid prolonging the working time of the vacuumizing system, thereby prolonging the service life of the machine. In addition, in the preheating stage, the heating system preheats the outer wall of the sterilization chamber, which can reduce the phenomenon of condensation of subsequently introduced water steam in a cold environment and improve the subsequent drying process.

In step 2, after the steam system introduces the high-temperature water steam into the sterilization chamber, and the sterilization chamber is vacuumized, the steam system introduces high-temperature water steam into the sterilization chamber again, and the sterilization chamber is vacuumized again, so as to completely empty the original air in the sterilization chamber. This can completely discharge the original air in the sterilization chamber as much as possible and improve the sterilization efficiency.

In step 5, an electromagnetic valve is arranged on the pipeline connecting the sterilization chamber to the vacuum system, so that the closing and opening of the pipeline are controlled by the electromagnetic valve.

The pressure detection device in step 6 includes a controller and a pressure sensor; the controller is electrically connected to the pressure sensor; the sterilization chamber is provided with an interface communicated to an inner cavity of the sterilization chamber; and the pressure sensor is connected to the interface.

A range of T1 in step 6 is 20 s to 2 min. A range of T2 in step 7 is 1 min to 5 min. As a pot body of the sterilizer may have different sizes, a value of P in step 7 may be a specific value in a range of 0.6 kPa to 2 kPa according to a specific situation.

The heating system includes a heating ring, a temperature detector, and a temperature control switch; the heating ring is arranged on the outer wall of the sterilization chamber to heat the sterilization chamber; the temperature detector is configured to detect the temperature inside the sterilization chamber and transmit a detected signal to the temperature control switch; and the temperature control switch is configured to turn on or turn off the heating ring. The steam system includes a water tank and a steam generator. The water and air discharge system and the vacuumizing system each include a vacuum pump, an exhaust pipe, an exhaust valve, a condenser, and a water-air separator which are arranged on the exhaust pipe; the condenser is configured to cool and condense steam passing through the exhaust pipe into water, and the water-air separator is configured to separate water in the exhaust pipe from air; the vacuum pump is communicated to an accommodating cavity through the exhaust pipe and is configured to extract condensate water and water steam from the sterilization chamber. An air return system includes an air filter, an air inlet pipe, and an inlet valve arranged on the air inlet pipe. The above components all belong to the prior art, such as various system mechanisms disclosed in the patent document, entitled sterilizer, No. CN111888492A. Therefore, detailed descriptions will not be made here.

The foregoing descriptions are merely specific implementations of the present disclosure, but are not intended to limit the protection scope of the present disclosure. Any variation or replacement easily figured out by a person skilled in the art within the technical scope disclosed in the present disclosure shall fall within the protection scope of the present disclosure. Therefore, the protection scope of the present disclosure shall be based on the protection scope of the claims.

## Claims

1. A drying determination method for a steam sterilizer, comprising the following steps:
step 1, in a drying stage, vacuumizing a sterilization chamber by a vacuumizing system, and when the sterilization chamber reaches a set vacuum degree, closing a pipeline connecting the sterilization chamber to the vacuumizing system;
step 2, in a drying detection stage, maintaining the sterilization chamber in vacuum for certain time T1, and during the maintaining the sterilization chamber in vacuum for certain time, continuously detecting a pressure in the sterilization chamber by a pressure detection device; and
step 3, in a drying determination stage, when the pressure detection device detects that the pressure in the sterilization chamber has increased by a value less than P during the period of time T1, determining that drying is completed, and turning off the vacuumizing system;
when the pressure detection device detects that the pressure in the sterilization chamber has increased by a value not less than P during the period of time T1, determining that drying is not completed; opening the pipeline connecting the sterilization chamber to the vacuumizing system again, continuing to vacuumize the sterilization chamber for certain time T2; and then returning to step 2 until it is determined that drying is completed.

2. The drying determination method for the steam sterilizer according to claim 1, wherein the pressure detection device in step 2 comprises a controller and a pressure sensor; the controller is electrically connected to the pressure sensor; the sterilization chamber is provided with an interface communicated to an inner cavity of the sterilization chamber; and the pressure sensor is connected to the interface.

3. The drying determination method for the steam sterilizer according to claim 1, wherein a range of T1 in step 2 is 20 s to 2 min.

4. The drying determination method for the steam sterilizer according to claim 1, wherein a range of T2 in step 3 is 1 min to 5 min.

5. The drying determination method for the steam sterilizer according to claim 1, wherein a value of P in step 3 is a specific value within a range of 0.6 kPa to 2 kPa.
